# EUROPEAN PATENT SPECIFICATION

(11) **EP 1 235 921 B1**
(45) Date of publication and mention of the grant of the patent: **14.03.2007**
(21) Application number: 00989221.7
(22) Date of filing: 06.12.2000
(51) Int. Cl.: C12N 15/82, A01H 4/00

(54) **SUGARBEET REGENERATION AND TRANSFORMATION**
REGENERIERUNG UND TRANSFORMATION VON ZUCKERRÜBEN
REGENERATION ET TRANSFORMATION DE BETTERAVES A SUCRE

(30) Priority: 07.12.1999 US 169512 P
(43) Date of publication of application: 04.09.2002
(73) Proprietor: Monsanto Technology LLC, St. Louis, Missouri 63167 (US)
(72) Inventor: CONNOR-WARD, Dannette, St. Charles, MO 63304 (US); HINCHEE, Maud, A., W., Summerville, SC 29483 (US)
(74) Representative: Helbing, Jörg
(86) International application number: PCT/US2000/033101
(87) International publication number: WO 2001/042480

(56) References cited:
- EP-A- 0 838 148
- WO-A-90/11682
- WO-A-99/23232
- LINDSEY K ET AL: "TRANSFORMATION OF SUGAR BEET BETA-VULGARIS BY AGROBACTERIUM-TUMEFACIENS" JOURNAL OF EXPERIMENTAL BOTANY, vol. 41, no. 226, 1990, pages 529-536, XP001020706 ISSN: 0022-0957 cited in the application
- JACQ BENOIT ET AL: "Factors influencing T-DNA transfer in Agrobacterium-mediated transformation of sugarbeet." PLANT CELL REPORTS, vol. 12, no. 11, 1993, pages 621-624, XP001020715 ISSN: 0721-7714 cited in the application
- BAJAJ-ED- Y P S: "Biotechnolgy in agriculture and forestry, Vol.23 Plant protoplasts and genetic engineering IV" BIOTECHNOLOGY IN AGRICULTURE AND FORESTRY, SPRINGER VERLAG, BERLIN, DE, vol. 23, 1993, pages 147-169, XP002083695 ISSN: 0934-943X
- D'HALLUIN K ET AL: "TRANSFORMATION OF SUGARBEET (BETA VULGARIS L.) AND EVALUATION OF HERBICIDE RESISTANCE IN TRANSGENIC PLANTS" BIO/TECHNOLOGY, MARTINSVILLE, US, vol. 10, 1 March 1992 (1992-03-01), pages 309-314, XP002056296
- JOERSBO M ET AL: "ANALYSIS OF MANNOSE SELECTION USED FOR TRANSFORMATION OF SUGAR BEET" MOLECULAR BREEDING: NEW STRATEGIES IN PLANT IMPROVEMENT, KLUWER ACADEMIC PUBLISHERS, NL, vol. 4, 1998, pages 111-117, XP000877300 ISSN: 1380-3743

## Description

### FIELD OF THE INVENTION

The invention relates to the preparation of genetically transformed sugarbeet plants. In particular, methods and materials for the transformation of sugarbeet plants by means of *Agrobacterium tumefaciens* mediated gene transfer are described.

### BACKGROUND OF THE INVENTION

Sucrose, commonly known as sugar, accounts for approximately 11% of human caloric intake worldwide. Sugarbeet (*Beta vulgaris*) is one of the two major agricultural sources of edible sugar. Approximately 40% of the sugar supply worldwide is derived from sugarbeets, with sugarcane, as the additional major agricultural source of sugar, accounting for nearly 60%. Countries in Europe and the former Soviet Union produce nearly all of their sugar from sugarbeet plants. In the United States, approximately 30% of consumed sugar is derived from sugarbeet.

Sugarbeet plants are susceptible to a range of pathogens and pests. Viral, fungal, bacterial, and insect damages can greatly reduce the yield and quality of harvested sugar. Enhancement of resistance to these factors has been slow by classical crossbreeding methods. Transformation of sugarbeet plants by modem molecular biology techniques is attractive, yet sugarbeets have proven to be highly recalcitrant to transformation until recently.

Lindsey and Jones (*Plant Cell Rep.,* 8: 71-74, 1989) describe the transformation of sugarbeet protoplasts by electroporation.

Catlin (*Plant Cell Rep.,* 9: 285-288, 1990) reports the effect of antibiotics on the inhibition of callus induction and plant regeneration from cotyledons of sugarbeet. Callus formation was found to be genotype dependent. Antibiotics were found to inhibit shoot formation. Catlin proposes that regeneration via cotyledonary explants combined with antibiotic screening protocols should prove useful in the selection of transformed sugarbeet callus.

Jacq et al. (*Plant Cell Rep.,* 12: 621-624, 1993) report the effects of genotype, acetosyringone, preculture, and coculture duration on the *Agrobacterium*-mediated transformation of sugarbeet. Hypocotyl and cotyledon explants were excised from seedlings and co-cultured with *Agrobacteria.* Transformants were quantitated by histochemical and fluorometric GUS assays. Transformed plants were not demonstrated.

Hall et al. (WO 95/10178) describe a method of transforming sugarbeet protoplasts by a polyethylene glycol procedure, followed by selection and regeneration. Transformation frequencies were between 2.5 x 10⁻⁵ and 6.1 x 10⁻⁴.

Lindsey and Gallois (*J. Exp. Botany,* 41: 529-536, 1990) describe the transformation of sugarbeet with binary vector--containing *Agrobacterium tumefaciens.* Shoot base tissue slices were inoculated by immersion in a liquid bacterial suspension, induced with 6-aminobenzylpurine to produce shoots, and selected by antibiotic resistance. Shoots were transferred to root induction medium containing 1-naphthaleneacetic acid and kanamycin. Little or no success was obtained in regenerating shoots from leaf tissues.

Hall et al. (*Nature Biotechnol.* 14: 1133-1138, 1996) describe a method of generating transgenic sugarbeet plants from protoplast stomatal guard cells. A polyethylene glycol DNA transformation was performed on protoplasts derived from stomatal guard cells. Selection of transformants was achieved by resistance to bialaphos. Stable transformation efficiencies for protoplasts were between 1.2 and 5.2 x 10⁻⁴. Protoplasts were regenerated into calli and plants.

Hayakawa et al. (*Proc. Jap. Sugar Beet Technol.* 36: 130-135, 1994) describe an *Agrobacterium*-mediated method for transformation of sugarbeets. Seeds were germinated, and the cotyledons removed. The cotyledons were grown until leaves appeared. Leaf pieces were cut, and transferred to medium to allow formation of shoots. The shoots were used to form explants, which were subsequently transformed with *Agrobacterium.* The transformed explants were allowed to form shoots, and subsequently rooted on appropriate media.

Fry et al. (Poster, Gordon Research Conference on Plant Cell and Tissue Culture, June 11-16, 1989) described the transformation and regeneration of sugarbeet cotyledon explants inoculated with *Agrobacterium tumefaciens* harboring exogenous nucleic acid sequences.

Despite the number of methods available for the transformation of sugarbeet, a need exists for a transformation method that is more efficient, provides a constant source of plant material for subsequent experiments, and provides an alternative to existing methods. The present invention uses leaf explants in an *Agrobacterium-*mediated transformation method that allows the remaining plant material to be propagated *in vitro* for future transformation experiments. The present invention provides improved methods for producing transgenic sugarbeet plants to impart desirable qualities by genetic engineering.

### SUMMARY OF THE INVENTION

In an effort to develop methods for the efficient transformation of sugarbeet plants, several steps were discovered to be greatly beneficial for improved results and experimental simplicity.

In one aspect of the present invention, leaf explants were found to be beneficial to subsequent transformation and such explants were readily prepared from the leaves of micropropagated shoots. Micropropagated cultures enable a scientist to maintain a continuous culture, significantly reducing the time involved in the preparation of transgenic sugarbeet plants. With micropropagated cultures being readily maintained, a scientist does not have to germinate seeds every time he or she begins a transformation procedure. Micropropagation is additionally beneficial in reducing or eliminating the occurrence of undesired chimeric tissues.

The features and details of the invention will be more fully appreciated in light of the following detailed description of the invention.

### DESCRIPTION OF THE FIGURES

The following figures form part of the present specification and are included to further demonstrate certain aspects of the present invention. The invention may be better understood by reference to one or more of these drawings in combination with the detailed description of specific embodiments presented herein.

| Figure | Description |
|---|---|
| 1 | Map of plasmid pMON 17303 |
| 2 | Map of plasmid pMON 13819 |
| 3 | Map of plasmid pMON 13835 |
| 4 | Map of plasmid pMON 13851 |
| 5 | Top view of micropropagated sugarbeet culture |
| 6 | Side view of micropropagated sugarbeet culture |

### DETAILED DESCRIPTION OF THE INVENTION

The present invention discloses a method for the efficient transformation of sugarbeet plants using *Agrobacterium tumefaciens* bacteria as the delivery agent of an exogenous nucleic acid sequence. The exogenous nucleic acid sequence is preferably in the form of a plant transformation vector. Any vector suitable for the transformation of plants can be used in the present invention. A suitable plant transformation plasmid or vector typically contains a selectable or screenable marker and associated regulatory elements as described, along with one or more nucleic acids capable of being expressed in a plant in a manner sufficient to confer a particular desirable trait or phenotype to the plant. Examples of suitable structural genes of interest envisioned by the present invention would include but are not limited to genes for insect or pest (bacterial, fungal, nematocidal) tolerance; herbicide tolerance; genes for quality improvements such as yield, nutritional enhancements, environmental or stress tolerances; or any desirable changes in plant physiology, growth, development, morphology, or plant product(s).

Alternatively, the DNA coding sequences can affect these phenotypes by encoding a non-translatable RNA molecule that causes the targeted inhibition of expression of an endogenous gene, for example via antisense- or cosuppression-mediated mechanisms (see, for example, Bird et al., Biotech. Gen. Engin. Rev. 9:207, 1991). The RNA could also be a catalytic RNA molecule (i.e., a ribozyme) engineered to cleave a desired endogenous mRNA product (see for example, Gibson and Shillitoe, Mol. Biotech. 7:125, 1997). Thus, any gene that produces a protein or mRNA that expresses a phenotype or morphology change of interest is useful for the practice of the present invention.

Exemplary nucleic acids that may be introduced by the methods encompassed by the present invention include for example, DNA sequences or genes from another species, or genes or sequences that originate with or are present in the same species, but are incorporated into recipient cells by genetic engineering methods rather than classical reproduction or breeding techniques. However, the term exogenous is also intended to refer to genes that are not normally present in the cell being transformed; or perhaps simply not present in the form, structure, etc., as found in the transforming DNA segment or gene; or genes that are normally present yet that one desires, e.g., to have over-expressed. Thus, the term "exogenous" gene or DNA is intended to refer to any gene or DNA segment that is introduced into a recipient cell, regardless of whether a similar gene may already be present in such a cell. The type of DNA included in the exogenous DNA can include DNA that is already present in the plant cell, DNA from another plant, DNA from a different organism, or a DNA generated externally, such as a DNA sequence containing an antisense message of a gene, or a DNA sequence encoding a synthetic or modified version of a gene.

Plant transformation vectors generally contain one or more nucleic acid coding sequences of interest under the transcriptional control of 5' and 3' regulatory sequences. Such vectors generally comprise, operatively linked in sequence in the 5' to 3' direction, a promoter sequence that directs the transcription of a downstream structural nucleic acid sequence in a plant; optionally, a 5' non-translated leader sequence; a nucleic acid sequence that encodes a protein of interest; and a 3' non-translated region that encodes a polyadenylation signal that functions in plant cells to cause the termination of transcription and the addition of polyadenylate nucleotides to the 3' end of the mRNA encoding the protein. The promoter may be homologous or heterologous to the structural nucleic acid sequence. Typical 5' to 3' regulatory sequences include a transcription initiation start site, a ribosome binding site, an RNA processing signal, a transcription termination site, and/or a polyadenylation signal. Plant transformation vectors also generally contain a selectable or screenable marker. Expression of the selectable or screenable marker sequence results in a phenotype that allows the differentiation of transgenic and non-transgenic sugarbeet tissues.

Any selectable marker suitable for plant transformation may be used in the present invention. Examples of selectable markers reported to be functional in plants include EPSPS (Klee et al., *Mol. Gen. Genet.* 210(3): 437, 1987), neomycin phosphotransferase (Loopstra et al., *Plant Mol. Biol.* 15(1): 1, 1990), hygromycin phosphotransferase (Meijer et al., *Plant Mol. Biol.* 16(5): 807, 1991), resistance to methotrexate (Irdani et al., *Plant Mol. Biol.* 37(6): 1079, 1998), phosphinothricin acetyl transferase (Shen et al., *J. Gen. Virol.* 76: 965, 1995), and chlorsulfuron resistance (Lee et al., *Plant Cell* 2(5): 415, 1990). Screenable markers reported to be functional in plants include GFP (Niwa et al., *Plant J.* 18(4): 455, 1999), GUS (Suzuki et al., *Plant Cell. Physiol.* 40(3): 289, 1999), CAT (Leisy et al., *Plant Mol. Biol.* 14(1): 41, 1990), and luciferase (Macknight et al., *Plant Mol. Biol.* 27(3): 457, 1995). In some cases, the structural nucleic acid coding sequence can also function as the selectable or screenable marker sequence.

In light of this disclosure, numerous other possible selectable or screenable marker genes, regulatory elements, and other sequences of interest will be apparent to those of skill in the art. Therefore, the foregoing discussion is intended to be exemplary rather than exhaustive.

In particular, the invention is directed towards a method for the preparation of transgenic sugarbeet plant the method comprising: selecting a sugarbeet seedling, the seedling comprising a cotyledon region and a hypocotyl region; removing the cotyledon region and upper half of the hypocotyl region from the seedling; contacting the cotyledon region and upper half of the hypocotyl region with micropropagation media to form a micropropagated shoot, the micropropagated shoot comprising at least one leaf or portion thereof including a leaf base wherein said leaf base comprises the bottom position of the leaf where the leaf is attached to the micropropagated shoot;
removing a leaf from the micropropagated shoot by separating the leaf at the leaf base; and contacting the leaf explant at the leaf base area with *Agrobacteria.* The leaf may be separated at the leaf base prior to inoculation with *Agrobacterium* by any number of methods, but preferably the leaf is cut at the leaf base using a suitable instrument such as a scalpel and the cut area of the leaf is contacted with the *Agrobacterium* culture. The explant is co-cultured with *Agrobacteria,* wherein the *Agrobacteria* comprise a vector, the vector comprising operatively linked in the 5' to 3' orientation: a promoter that directs transcription of a structural nucleic acid sequence; a structural nucleic acid sequence; and a 3' transcription terminator, and after incubation with *Agrobacterium*, the infected leaf explant comprises a transgenic sugarbeet cell.

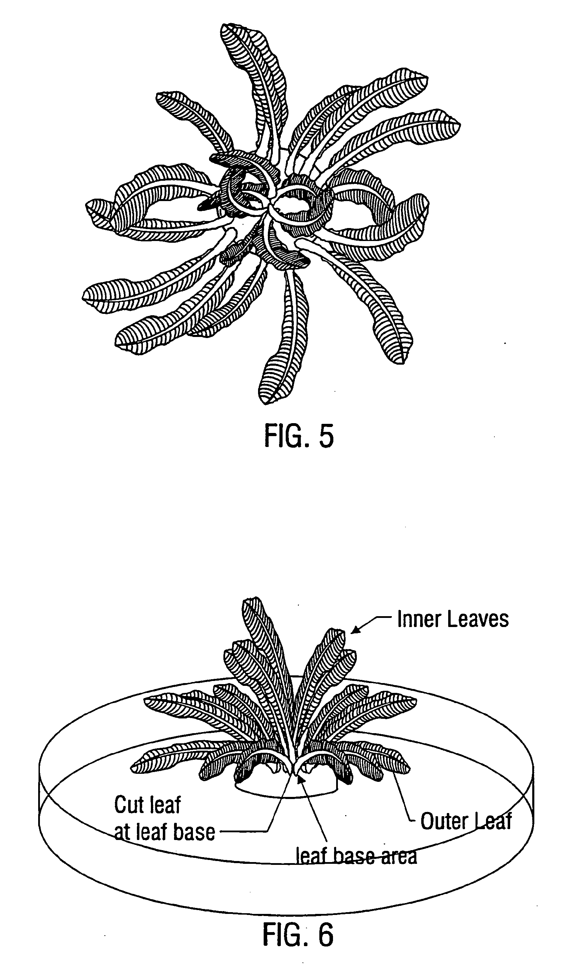

The leaf explant comprises the leaf base herein defined as the lower portion of the leaf where the leaf contacts the base region of the explant (see Figures 5 and 6 for illustrations of the micropropagated sugarbeet culture). Any leaf comprising a leaf base can be used in the present invention. The entire leaf or any portion thereof comprising the leaf base may be used. Preferably, the leaf is excised at the leaf base and the intact leaf comprising the leaf base is used for the transformation process. The leaf base comprises the bottom portion of the leaves where the leaves are attached to the micropropagated shoot. The leaf explant thus includes any leaf or portion of a leaf that includes the leaf base. Accordingly, the leaf explant can include but is not limited to the lower 10% of the leaf, the bottom 25%, 50%, or the entire leaf or portions thereof, all of which comprise a leaf base. The micropropagated sugarbeet culture comprises multiple layers of leaves that are attached at the leaf base. Accordingly, a leaf in any layer including any inner or outer leaf comprising a leaf base can be used (see Figures 5 and 6). Preferably, the outermost leaves are selected such that the innermost leaf layer is left intact. The remaining leaves can be micropropagated or stored and grown *in vitro.* The remaining leaves and newly emerged leaves can later be used as an explant source for future experiments.

The *Agrobacteria* may generally be any *Agrobacteria,* and preferably is *Agrobacterium tumefaciens.* Preferred strains would include but are not limited to *Agrobacterium tumefaciens* strain C58, a nopaline-type strain that is used to mediate the transfer of DNA into a plant cell, octopine-type strains such as LBA4404 or succinamopine-type strains, e.g., EHA101 or EHA105. The use of these strains for plant transformation has been reported and the methods are familiar to those of skill in the art. The structural nucleic acid sequence can be any transcribable sequence that confers a desirable property to the plant. Preferably, the structural nucleic acid sequence encodes a protein or an antisense RNA sequence.

An alternative embodiment is directed towards methods for the preparation of transgenic sugarbeet plant from a micropropagation culture. A micropropagation culture is grown to form a micropropagated shoot, the micropropagated shoot comprising at least one leaf explant including a leaf base; removing a leaf at the leaf base from the micropropagated shoot: and contacting the leaf explant at the leaf base area with *Agrobacteria* wherein: the *Agrobacteria* comprise a vector, the vector comprising operatively linked in the 5' to 3' orientation: a promoter that directs transcription of a structural nucleic acid sequence; a structural nucleic acid sequence; and a 3' transcription terminator. After incubation with *Agrobacterium* the infected leaf explant comprises a transgenic sugarbeet cell. The *Agrobacteria* may generally be any *Agrobacteria,* and preferably is *Agrobacterium tumefaciens.* The structural nucleic acid sequence can be any transcribable sequence that confers a desirable property to the plant. Preferably, the structural nucleic acid sequence encodes a protein or an antisense RNA sequence.

As set forth above, the invention provides a method for the preparation of transgenic sugarbeet plants. The method: preferably comprises selecting a sugarbeet seedling, the seedling comprising a cotyledon region and a hypocotyl region; removing the cotyledon region and upper half of the hypocotyl region from the seedling; contacting the cotyledon region and upper half of the hypocotyl region with micropropagation media to form a micropropagated shoot, the micropropagated shoot comprising at least one leaf comprising a leaf base; removing a leaf from the micropropagated shoot at the leaf base; contacting the leaf with *Agrobacteria* at the leaf base area to form an infected leaf wherein: the *Agrobacteria* comprise a vector, the vector comprising operatively linked in the 5' to 3' orientation: a promoter that directs transcription of a structural nucleic acid sequence, a structural nucleic acid sequence, and a 3' transcription terminator; culturing the infected leaf explant to form a transgenic shoot; culturing the transgenic shoot to form a transgenic rooted shoot; and growing the transgenic rooted shoot to form a transgenic sugarbeet plant. The *Agrobacteria* may generally be any *Agrobacteria,* and preferably is *Agrobacterium tumefaciens.* The structural nucleic acid sequence can be any transcribable sequence that confers a desirable property to the plant. Preferably, the structural nucleic acid sequence encodes a protein or an antisense RNA sequence. The sugarbeet seed may be sterilized by any acceptable method, and preferably by contacting the sugarbeet seed with sodium hydroxide, sodium hypochlorite, ethanol, or captan. The sugarbeet seed is preferably germinated in the dark or under low light conditions. The micropropagation media may generally be any micropropagation media accepted in the art. Examples of suitable media would include but are not limited to MS-based media (Murashige and Skoog, Physiol. Plant, 15:473, 1962) or N6-based media (Chu et al., Scientia Sinica, 18:659, 1975) supplemented with media additives that can include but are not limited to amino acids, macroelements, iron, microelements, vitamins and organics, carbohydrates, undefined media components such as casein hydrolysates, an appropriate gelling agent such as a form of agar, such as a low melting point agarose or Gelrite if desired. Those of skill in the art are familiar with the variety of tissue culture media, which when supplemented appropriately, support plant tissue growth and development and are suitable for plant transformation and regeneration. These tissue culture media can either be purchased as a commercial preparation, or custom prepared and modified. The media selected can be supplemented with appropriate additives depending on the stage in the transformation process. For example, for micropropagation a media herein referred to as micropropagation media that supports the growth and maintenance of plant tissue *in vitro* can be used. Accordingly, in other stages of the transformation process including but not limited to germination, shoot induction, and root induction, media supplemented with appropriate additives are used and can include but are not limited to media such as Linsmaier and Skoog (Linsmaier and Skoog, Physiol. Plant., 18:100, 1965), Uchimiya and Murashige (Uchimiya and Murashige, Plant Physiol. 54:936, 1974), Gamborg's media (Gamborg et al., Exp. Cell Res., 50:151, 1968), McCown's Woody Plant Media (McCown and Loyd, Hort. Science, 16:453, 1981), Nitsch and Nitsch (Nitsch and Nitsch, Science 163:85-87, 1969), and Schenk and Hildebrandt (Schenk and Hildebrandt, Can J. Bot. 50:199, 1972) or derivations of these media supplemented accordingly. Those of skill in the art are aware that media and media supplements such as nutrients and growth regulators for use in various stages of the plant maintenance, and transformation and regeneration stages in addition to other culture conditions such as light intensity during incubation, pH, and incubation temperatures can be optimized for a particular plant variety.

The following experimental protocol is illustrative of the invention. One of ordinary skill will appreciate that various modifications in materials, media, duration of processes, temperature, and lighting conditions may be made.

### Seed sterilization

Seeds may be sterilized using any acceptable method. The following method has been found to be effective.

Sugarbeet seeds are added to a solution containing approximately 1 N sodium hydroxide and are stirred for about one hour at room temperature. The liquid is decanted, and the seeds rinsed with distilled water to remove residual sodium hydroxide. Cold water is added, and the seeds are stirred for about one hour. The seeds are removed from the liquid, and a small portion of the seed coat from each embryo is removed to expose a portion of the seed embryo. The seeds are rinsed briefly with 95% ethanol. The ethanol solution is decanted, and the seeds are stirred in a dilute 2.5% w/v sodium hypochlorite solution for about twenty minutes. The liquid is removed, and the seeds are rinsed in sterile distilled water three times. Captan (a wettable powder used as an aqueous solution for the control of certain fungal diseases, Micro Flo Co., Lakeland, FL) and a small amount of sterile water sufficient to cover the seeds is added. The seeds are soaked for at least one hour. Soaking may be continued overnight if desired.

### Seed germination

Seeds may be germinated using any acceptable method. The following method has been found to be effective. Other media formulations including but not limited to those described earlier may be suitable to promote germination in place of the media formulations described below.

Seeds are transferred from the captan suspension to plates containing MSMO medium (Table 1). Approximately 15 seeds are placed on each 100 X 25 mm germination plate. The plates are placed in bags, preferably plastic bags lacking holes. Preferably the seeds are germinated in the dark and the bags are placed in the dark at approximately 20°C-25°C, preferably at approximately 23°C-24°C. The incubation in the dark can be done in any manner including but not limited to placing the bags in a sealed cardboard box. The optimal duration of storage in the dark varies by sugarbeet genotype, but will typically vary between two and four weeks. The seed coat is carefully removed from the germinated seedlings, and the seedlings are subsequently transferred to containers holding MSMO medium. The seedlings are incubated at approximately 23°C-24°C for about 1-5 days, preferably about 2 days. The photoperiod during incubation is maintained at an approximately 16:8 light:dark cycle.

**Table 1: MSMO medium**

| Component | Concentration |
|---|---|
| MSMO Salts (Sigma M-6899) | 4.4 g/L |
| Sucrose | 30 g/L |
| Phytagar | 7 g/L |

### Initiation and maintenance of micropropagated shoot cultures

Micropropagated shoot cultures may be initiated using any acceptable method. The following method has been found to be effective in the laboratory. Other media formulations as described earlier may be suitable to initiate micropropagated shoot cultures in place of the media formulations described below.

The lower half of the hypocotyl region is carefully removed from the germinated seedlings. The portion of the seedlings containing cotyledons and the upper portion of the hypocotyl is transferred to MSMO medium containing 0.1 mg/L 6-benzylaminopurine. After three weeks incubation with lighting as above, the shoots are transferred to fresh medium. After an additional three weeks, the leaves may be used for transformation. Preferably, the outermost leaves are used and the innermost leaves are used to maintain the tissue *in vitro.* For maintenance of the micropropagated cultures, after the leaves are removed for subsequent transformation, the apical and inner-most leaf region (generally about 0.5-3.0 cm) is transferred to fresh medium to maintain the micropropagated shoot cultures (see Figures 5 and 6). Figure 5 illustrates a top view of the sugarbeet micropropagation culture. Figure 6 illustrates a side view that depicts outer leaves, inner leaves, the leaf base area, and the cutting or wounding area where the leaves are removed at the leaf base. Sugarbeet micropropagation cultures preferably are transferred to fresh medium at least once per month to maintain healthy cultures and a ready source of tissue for transformation. By maintaining the sugarbeet tissue in this manner, suitable explant material for transformation is constantly available without the need to initiate cultures from seeds, saving at least a month in the process.

### Preparation of bacterial cultures

Bacterial cultures may be prepared using any acceptable method. The following method has been found to be effective in the laboratory. Other media formulations may be suitable to prepare bacterial cultures in place of the media formulations described below. Any *Agrobacterium* strain suitable for participation in the inventive methods may be used, and *Agrobacterium tumefaciens* is preferred. Methods for the growth and maintenance of *Agrobacterium* cultures are known to those of skill in the art.

*Agrobacterium tumefaciens* containing the exogenous DNA sequence to be transferred to sugarbeet are typically plated on LB medium containing selective antibiotics (see for example LBsck medium supplemented with spectinomycin, chloramphenicol, and kanamycin, Table 2). The bacteria are grown at room temperature for about two to three days. The bacteria are used to initiate liquid cultures in LBsck two days prior to transformation in order to achieve proper cell density. Bacteria are diluted approximately 100 times into ABsck (Table 3) or YEP (Table 4) medium. Approximately 2 mL of medium is used per culture, although one skilled in the art will recognize that varying the scale of the culture will not significantly impact the growth of the bacteria. The cultures are allowed to incubate overnight on an orbital shaker. The bacterial cultures are diluted approximately 12.5 X into ABsck medium or ABsck medium supplemented with 200 µM acetosyringone, preferably with a total volume of about 50 mL. Cultures are placed on an orbital shaker and incubated at about 26°C overnight. The culture is monitored until it reaches a cell density of about 3.0 X 10⁹ cells/mL, which corresponds to an OD₆₀₀ of about 1. The volume is adjusted with TXD medium (Table 5) to give a cell density of about 1 X 10⁹ cells/mL.

**Table 2: LBsck medium**

| Component | Concentration |
|---|---|
| Peptone | 10 g/L |
| Yeast extract | 5 g/L |
| Sodium chloride | 5 g/L |
| Difco bacto agar | 15 g/L |
| Spectinomycin | 100 mg/L |
| Chloramphenicol | 25 mg/L |
| Kanamycin | 50 mg/L |

**Table 3: ABsck medium**

| Component | Concentration |
|---|---|
| Glucose | 5 g/L |
| Phytagar | 7.5 g/L |
| AB salts | 25 mL/L |
| AB buffer | 25 mL/L |
| Spectinomycin | 0.1 mg/L |
| Chloramphenicol | 0.025 mg/L |
| Kanamycin | 0.05 mg/L |

| | |
|---|---|
| AB Salts (500 mL): 10 g NH₄Cl, 12.5 g MgSO₄•7H₂O. 1.5 g KCl, 0.1 g CaCl₂ or 0.132 g CaCl₂•2H₂O, 25 mg FeSO₄ or 45.55 mg FeSO₄•7 H₂O. AB Buffer (500 mL): 39.33 g K₂HPO₄•3H₂O or 30.0 g K₂HPO₄, 11.5 g NaH₂PO₄• 2H₂O. | |

**Table 4: YEP medium**

| Component | Concentration |
|---|---|
| Yeast extract | 10 g/L |
| Peptone | 10 g/L |
| Sodium chloride | 5 g/L |
| pH=7 | |

**Table 5: TXD medium**

| Component | Concentration |
|---|---|
| MS salts with B5 vitamins (Sigma M0404) | 4.4 g/L |
| PCPA | 4 mg/L |
| Kinetin | 5 µg/L |
| Sucrose | 30 g/L |
| pH adjusted to 5.8 | |

### Preparation of co-culture plates

Co-culture plates may be prepared using any acceptable media formulation. Media formulations other than that described below may be suitable.

Plates are poured containing 1/10 MS medium (Table 6) preferably supplemented with 200 µM acetosyringone and 1 mM galacturonic acid. The acetosyringone and galacturonic acid are added to the MS medium after autoclaving. A sterile filter paper is placed on top of the solid medium and moistened with TXD medium prior to use with explants.

**Table 6: MS medium**

| Component | Concentration |
|---|---|
| MS Salts (Sigma) | 4.4 g/L |
| Sucrose | 30 g/L |
| SB Vitamin #1 | 2 mL/L |
| SB Vitamin #2 | 1 mL/L |
| pH = 5.95 | |

### Leaf base explanting

Explanting may be performed using any acceptable method. The following method has been found to be effective. Other media formulations as described earlier may be suitable for explanting in place of the media formulations described below.

Leaves from the micropropagated shoots are excised and transferred to a petri dish containing sterile filter paper moistened with TXD medium. Leaves from any part of the micropropagated shoot can be used in the present invention. Accordingly, any inner or outer leaf can be used for subsequent transformation. Preferably the outermost leaves are used. The leaf includes the leaf blade and leaf base and can be excised in any manner. Preferably the leaves are removed by cutting the leaf at the leaf base (see Figure 6) such that the leaf is primarily intact and the wound site is at the leaf base. After excising about 25 leaves from the micropropagated shoots, the explants are transferred to the diluted bacterial culture and incubated. The duration of the incubation of the leaf explants with *Agrobacterium* can occur for any length of time. Preferably the incubation period with *Agrobacterium* is up to sixty minutes, more preferably about fifteen to twenty minutes and the incubation is performed in a manner that exposes the wound site to the *Agrobacterium* culture. Preferably the lower wounded site of the leaf explant at the leaf base is in contact with the *Agrobacterium* culture. The explants are removed from the bacterial culture, blotted on sterile filter paper such as Whatman filter paper, and transferred to co-culture plates.

The co-culture period can occur for any length of time. Preferably the co-culture duration is up to five days. More preferably, the co-culture period is from about two to five days. Even more preferably, the co-culture period is four days. The co-culture plates are placed in sealed plastic bags and incubated for about four days at approximately 22°C-24°C. A photoperiod of about 16:8 light:dark is used for the incubation period.

Optionally, the leaves from the micropropagated shoots may be excised as described above, and transferred directly to co-culture plates for an overnight preculture period at about 22°C-24°C prior to addition of the bacterial culture. Although overnight preculture is preferred, preculturing for at least about six hours is also effective. After the preculture period, the explants are infected with *Agrobacterium* by adding the bacterial suspension directly to the explants on the co-culture plates. After an incubation of about twenty minutes, the bacteria can be removed by any manner including aspiration and the explants can remain on the same plates for the duration of the co-culture period.

### Culture and selection conditions

Explants may be cultured and selected using any acceptable method. The following method has been found to be effective. Other media formulations as described earlier may be suitable for culture and selection in place of the media formulations described below.

Explants are transferred from co-culture plates to a culture medium. Preferably, the culture media is an MS-based medium supplemented with 0.5 mg/L benzylaminopurine (BAP) or benzyl adenine (BA), 0.05 mg/L 1-naphthaleneacetic acid, 500 mg/L carbenicillin, and 100 mg/L cefotaxime. The co-culture conditions can vary depending on the selectable marker. For glyphosate (N-phosphonomethylglycine) selection, the infected explants are placed on a delay medium without the addition of the selective agent glyphosate. The length of the delay period will depend on the genotype used. Experiments are performed with each genotype to determine the optimal duration of the delay period. Typically, the delay period can occur for up to ten days. Preferably the delay period without selection is for about two to seven days. Even more preferably, the delay period without selection is for about six days at approximately 24°C preferably in plastic bags with holes. Experiments are performed with each genotype to determine the optimal level of the selective agent. For glyphosate selection, after the delay period, the infected explants are transferred from the delay medium (without glyphosate) to an MS-based medium supplemented with 0.50 mg/L benzyladenine, 0.05 mg/L 1-naphthaleneacetic acid, 500 mg/L carbenicillin, 100 mg/L cefotaxime, and 0.05-0.10 mM glyphosate (preferably about 0.075 mM glyphosate). Explants can be cultured for about two to four weeks on the selection medium. Preferably, explants are cultured for about four three weeks at about 24°C, preferably in plastic bags with holes. A photoperiod of about 16:8 light:dark is used for the culture period. Explants are transferred to the same medium containing the appropriate selection agent every three weeks. Generally, two to three transfers are sufficient to identify a transgenic plant.

### Micropropagation and rooting procedures

Micropropagation and rooting may be performed using any acceptable method. The following method has been found to be effective. Other media formulations as described earlier may be suitable for micropropagation and rooting in place of the media formulations described below.

Micropropagation is beneficial to reduce or eliminate undesired chimeric tissues. The use of micropropagation is especially helpful for obtaining the desired transgenic plants and saves time by eliminating the need to obtain plant material starting from seeds. The tissue can be maintained in a number of tissue culture vessels including but not limited to petri plates or sundae cups.

Transformed shoots are transferred to micropropagation medium containing MS-based medium supplemented with 0.5 mg/L 6-benzyladenine, 500 mg/L carbenicillin, and 100 mg/L cefotaxime. Shoots are grown and divided on this medium until the desired number of shoots has been obtained. Preferably, for glyphosate selection, the glyphosate can be added to the micropropagation medium to aid in the selection of clonal material. By clonal material as used herein is meant that the entire shoot is transformed and does not contain any chimeric plant material that consists of both transformed and nontransformed tissue. Individual shoots are rooted on ½ MS medium supplemented with 5 mg/L indolebutyric acid, 100 mg/L cefotaxime, and 500 mg/L carbenicillin. Roots should appear in approximately 4-12 weeks. Rooted shoots can be transplanted to soil, preferably Metro Mix 350 (Metro Mix is a registered trademark of Scotts Company, Marysville, OH).

The following examples are included to demonstrate preferred embodiments of the invention. It should be appreciated by those of skill in the art that the techniques disclosed in the examples that follow represent techniques discovered by the inventor to function well in the practice of the invention, and thus can be considered to constitute preferred modes for its practice. However, those of skill in the art should, in light of the present disclosure, appreciate that many changes can be made in the specific embodiments that are disclosed and still obtain a like or similar result without departing from the spirit and scope of the invention.

### EXAMPLES

### Example 1: Plasmid constructs

All constructs described in the Examples are double border plant transformation vectors containing an *E. coli* origin of replication (ori322), a broad host range origin of replication (ori-V), and a coding region (Spc/Str) for Tn7 adenyltransferase, which confers resistance to spectinomycin and streptomycin. For plant transformation, the host bacterial strain used was *Agrobacterium tumefaciens* strain ABI. The promoter, coding region, and 3' terminators are indicated in parentheses.

P-e35S is the promoter for 35S RNA from CaMV containing a duplication of the -90 to -300 region as described in U. S. Patent No. 5,424,200; P-FMV is the 35S promoter from the Figwort Mosaic Virus as described in U. S. Patent No. 5,378,619; P-aa-chit is the promoter for *Arabidopsis* acidic chitinase as described in Samac and Shah (Plant Cell 3:1063, 1991); AEPSPS/CTP2 is the transit peptide region of *Arabidopsis* EPSPS (5-enolpyruvyl-3-phosphoshikimic acid synthase) as described in U. S. Patent No. 5,633,435; CP4syn is the coding region for CP4 EPSPS (synthetic sequence) as described in U. S. Patent No. 5,633,435; fbp is the coding sequence for *E. coli* fructose 1,6-bisphosphatase as described in Hamilton et al. (*Nucleic Acids Res.* 16: 8707, 1988); glgC16 is an *E. coli* ADP glucose phosphorylase gene as described in U. S. Patent No. 5,648,249; mz SPS is the coding region for maize sucrose phosphate synthase as described in U. S. Patent No. 5,750,869; E9 3' is the 3' end of E9 pea rbc gene, which functions as a polyadenylation signal; 7S is the soybean 7S seed storage protein gene 3' termination signal; GUS:1 is the beta-glucuronidase gene coding sequence from *E*. *coli* (Jefferson, R.A., *Proc. Natl. Acad. Sci. U.S.A.* 83: 8447-8451, 1987) modified to contain an NcoI restriction site (CCATGG) at the start codon.

### Example 2: Transformation with ABI pMON 17303

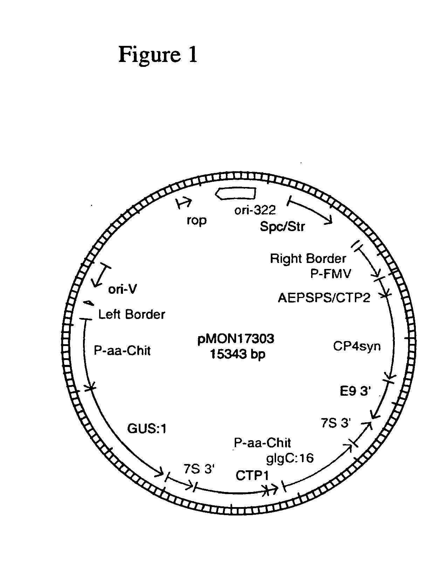

The outermost leaves were excised directly from micropropagated sugarbeet shoots and used as explants for transformation. The leaves were cut at the leaf base and incubated with *Agrobacterium* such that the leaf base area was in contact with the *Agrobacterium* culture. The *Agrobacterium tumefaciens* strain ABI contained the the construct pMON17303 (P-e35S/mz SPS/E9 3', P-FMV/CP4syn/E9 3'; Figure 1) and the transformation method was as described in the Detailed Description of the Invention. No preculture was used in the transformations using this construct. The selection of transformed cells was performed using 0.075 mM glyphosate. From 573 explants, 3 transformed plants were obtained for a transformation efficiency of 0.5%.

### Example 3: Transformation with construct ABI pMON 13819

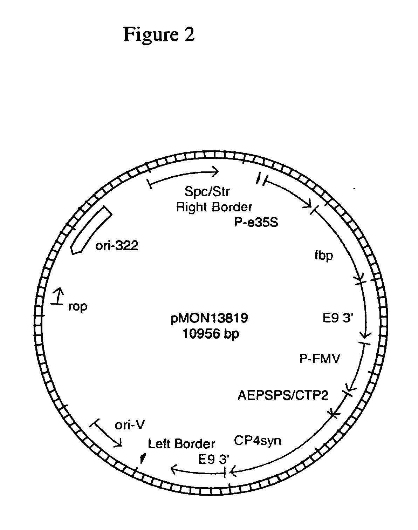

The outermost leaves were excised directly from micropropagated sugarbeet shoots and used as explants for transformation. The leaves were cut at the leaf base and incubated with *Agrobacterium* such that the leaf base area was in contact with the *Agrobacterium* culture. The *Agrobacterium tumefaciens* strain ABI contained the construct pMON13819 (P-e35S/fbp/E9 3', P-FMV/CP4syn/E9 3'; Figure 2). The transformation and selection was performed as described in Example 2 and in the Detailed Description of the Invention. From 1875 explants, 52 transformed plants were obtained for a transformation efficiency of 3%.

### Example 4: Transformation with construct ABI pMON13835

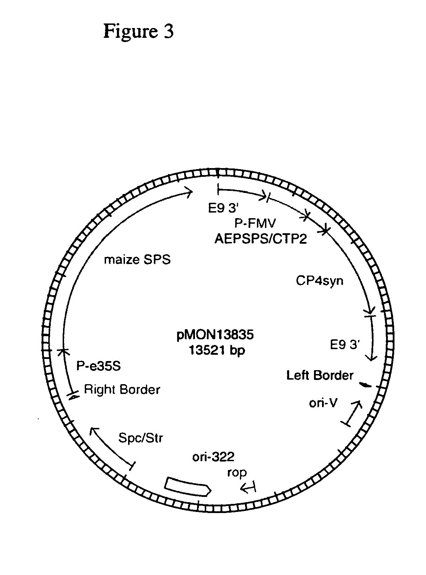

The outermost leaves were excised directly from micropropagated sugarbeet shoots and used as explants for transformation. The leaves were cut at the leaf base and incubated with *Agrobacterium* such that the leaf base area was in contact with the *Agrobacterium* culture. The *Agrobacterium tumefaciens* strain ABI contained the construct pMON13835 (P-e35S/mz SPS/E9 3', P-FMV/CP4syn/E9 3'; Figure 3). The transformation and selection was performed as described in Example 2 and in the Detailed Description of the Invention. From 2580 explants, 12 transformed plants were obtained for a transformation efficiency of 0.5%.

### Example 5: Transformation with construct ABI pMON13851

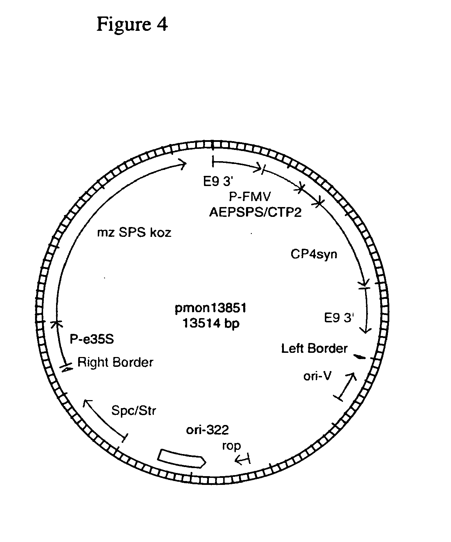

The outermost leaves were excised directly from micropropagated sugarbeet shoots and used as explants for transformation. The leaves were cut at the leaf base and incubated with *Agrobacterium* such that the leaf base area was in contact with the *Agrobacterium* culture. The *Agrobacterium tumefaciens* strain ABI contained the construct pMON13851 (P-e35S/mzSPS/E93'/P-FMV/CP4syn/E93'; Figure 4). The transformation and selection was performed as described in Example 2 and in the Detailed Description of the Invention, with the addition of an overnight preculturing step prior to contacting the plant tissue with *Agrobacteria.* From 2890 explants, 14 transformed plants were obtained for a transformation efficiency of 0.5%.

## Claims

1. A method for the preparation of a transgenic sugarbeet plant, the method comprising:
selecting a sugarbeet seedling, the seedling comprising a cotyledon region and a hypocotyl region;
removing the cotyledon region and upper half of the hypocotyl region from the seedling;
contacting the cotyledon region and upper half of the hypocotyl region with micropropagation media to form a micropropagated shoot, the micropropagated shoot comprising at least one leaf or portion thereof comprising a leaf base, wherein said leaf base comprises the bottom position of the leaf where the leaf is attached to the micropropagated shoot;
removing a leaf from the micropropagated shoot at the leaf base;
contacting the leaf base with *Agrobacteria* in a manner forming a tissue comprising a transgenic sugarbeet cell;
culturing said tissue to form a transgenic shoot;
culturing the transgenic shoot to form a transgenic rooted shoot; and
growing the transgenic rooted shoot to form a transgenic sugarbeet plant capable of expressing an exogenous structural nucleic acid sequence,
wherein:
the *Agrobacteria* comprise a vector; and
the vector comprises operatively linked in the 5' to 3' orientation: a promoter that directs transcription of an exogenous structural nucleic acid sequence; an exogenous structural nucleic acid sequence; and a 3' transcription terminator.

2. The method of claim 1, wherein the leaf or portion thereof comprises an outer leaf or portion thereof

3. The method of claim 1, wherein the leaf or portion thereof comprises an inner leaf or portion thereof.

4. The method of claim 1, wherein the sugarbeet seed is germinated in the dark.

5. The method of claim 1, wherein the micropropagation media comprises MSMO media and about 0.1 mg/L 6-aminobenzylpurine.

6. The method of claim 1, wherein the selection agent comprises glyphosate.

7. The method of claim 1, wherein the *Agrobacteria* is *Agrobacterium tumefaciens.*

8. The method of claim 1, wherein the structural nucleic acid sequence encodes a protein or an antisense RNA sequence.

9. The method of claim 1, wherein the leaf or portion thereof comprising a leaf base is precultured prior to the contacting step with *Agrobacteria.*

## Patentansprüche

1. Verfahren zur Herstellung einer transgenen Zuckerrübenpflanze, wobei das Verfahren Folgendes umfasst:
Auswählen eines Zuckerrübensämlings, wobei der Sämling einen Keimblattbereich und einen Hypokotylbereich umfasst;
Entfernen des Keimblattbereichs und der oberen Hälfte des Hypokotylbereichs von dem Sämling;
In-Kontakt-Bringen des Keimblattbereichs und der oberen Hälfte des Hypokotylbereichs mit einem Mikropropagationsmedium unter Bildung eines mikropropagierten Sprosses, wobei der mikropropagierte Spross wenigstens ein Blatt oder ein Teil davon, der eine Blattbasis umfasst, umfasst, wobei die Blattbasis die untere Position des Blattes umfasst, wo das Blatt an dem mikropropagierten Spross befestigt ist;
Entfernen eines Blatts aus dem mikropropagierten Spross an der Blattbasis;
In-Kontakt-Bringen der Blattbasis mit Agrobakterien, so dass ein Gewebe entsteht, das eine transgene Zuckerrübenzelle umfasst;
Kultivieren des Gewebes unter Bildung eines transgenen Sprosses;
Kultivieren des transgenen Sprosses unter Bildung eines transgenen bewurzelten Sprosses; und
Wachsenlassen des transgenen bewurzelten Sprosses unter Bildung einer transgenen Zuckerrübenpflanze, die eine exogene strukturelle Nucleinsäuresequenz exprimieren kann, wobei:
die Agrobakterien einen Vektor umfassen; und
der Vektor in 5'→3'-Orientierung funktionell verknüpft Folgendes umfasst: einen Promotor, der die Transcription einer exogenen strukturellen Nucleinsäuresequenz steuert; eine exogene strukturelle Nucleinsäuresequenz; und einen 3'-Transcriptionsterminator.

2. Verfahren gemäß Anspruch 1, wobei das Blatt oder das Teil davon ein äußeres Blatt oder ein Teil davon umfasst.

3. Verfahren gemäß Anspruch 1, wobei das Blatt oder das Teil davon ein inneres Blatt oder ein Teil davon umfasst.

4. Verfahren gemäß Anspruch 1, wobei der Zuckerrübensämling im Dunkeln keimen gelassen wird.

5. Verfahren gemäß Anspruch 1, wobei das Mikropropagationsmedium MSMO-Medium und etwa 0,1 mg/l 6-Aminobenzylpurin umfasst.

6. Verfahren gemäß Anspruch 1, wobei das Selektionsmittel Glyphosat umfasst.

7. Verfahren gemäß Anspruch 1, wobei es sich bei den Agrobakterien um *Agrobacterium tumefaciens* handelt.

8. Verfahren gemäß Anspruch 1, wobei die strukturelle Nucleinsäuresequenz ein Protein oder eine Antisense-RNA-Sequenz codiert.

9. Verfahren gemäß Anspruch 1, wobei das Blatt oder sein Teil, das eine Blattbasis umfasst, vor dem Schritt des In-Kontakt-Bringens mit Agrobakterien vorkultiviert wird.

## Revendications

1. Procédé de préparation d'un plant transgénique de betterave sucrière, le procédé comprenant :
la sélection d'une plantule de betterave sucrière, la plantule comprenant une région de cotylédons et une région de l'hypocotyle ;
l'élimination de la région de cotylédons et de la moitié supérieure de la région de l'hypocotyle de la plantule ;
la mise en contact de la région de cotylédons et de la moitié supérieure de la région de l'hypocotyle avec les milieux de micropropagation pour former une pousse micropropagée, la pousse micropropagée comprenant au moins une feuille ou une portion de celle-ci comprenant une base de feuille, dans laquelle ladite base de feuille comprend la position du bas de la feuille où la feuille est fixée à la pousse micropropagée ;
l'élimination d'une feuille de la pousse micropropagée au niveau de la base de la feuille ;
la mise en contact de la base de la feuille avec *Agrobacteria* d'une manière formant un tissu comprenant une cellule transgénique de betterave sucrière ;
la culture dudit tissu pour former une pousse transgénique ;
la culture de la pousse transgénique pour former une pousse transgénique enracinée ; et
la croissance de la pousse transgénique enracinée pour former un plant transgénique de betterave sucrière capable d'exprimer une séquence exogène structurelle d'acide nucléique, dans laquelle :
l'*Agrobacteria* comprend un vecteur ; et
le vecteur comprend fonctionnellement lié dans le sens 5' à 3' : un promoteur qui dirige la transcription d'une séquence exogène structurelle d'acide nucléique ; une séquence exogène structurelle d'acide nucléique ; et un codon de terminaison de la transcription 3'.

2. Procédé selon la revendication 1, dans lequel la feuille ou la portion de celle-ci comprend une feuille ou une portion externe de celle-ci.

3. Procédé selon la revendication 1, dans lequel la feuille ou la portion de celle-ci comprend une feuille interne ou une portion de celle-ci.

4. Procédé selon la revendication 1, dans lequel la graine de betterave sucrière est mise à germer à l'obscurité.

5. Procédé selon la revendication 1, dans lequel les milieux de micropropagation comprennent des milieux MSMO et environ 0,1 mg/L de 6-aminobenzylpurine.

6. Procédé selon la revendication 1, dans lequel l'agent de sélection comprend du glyphosate.

7. Procédé selon la revendication 1, dans lequel l'agrobactérie est *Agrobacterium tumefaciens.*

8. Procédé selon la revendication 1, dans lequel la séquence structurelle d'acide nucléique code une protéine ou une séquence d'ARN antisens.

9. Procédé selon la revendication 1, dans lequel la feuille ou la portion de celle-ci comprenant une base de feuille est précultivée avant l'étape de mise en contact avec *Agrobacteria.*
